(19) Europäisches Patentamt
European Patent Office
Office européen des brevets

(11) **EP 2 752 158 A1**

(12) **EUROPEAN PATENT APPLICATION**
published in accordance with Art. 153(4) EPC

(43) Date of publication:
**09.07.2014 Bulletin 2014/28**

(21) Application number: **12828933.7**

(22) Date of filing: **29.08.2012**

(51) Int Cl.:
***A61B 5/053*** (2006.01)

(86) International application number:
**PCT/ES2012/070635**

(87) International publication number:
**WO 2013/030425 (07.03.2013 Gazette 2013/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2011 ES 201131426**

(71) Applicant: **Universidad De Extremadura 06071 Badajoz (ES)**

(72) Inventors:
• **AUSIN SANCHEZ, Jose, Luis E-06071 Badajoz (ES)**

• **RAMOS MAGANES, Javier E-06071 Badajoz (ES)**
• **DUQUE CARRILLO, Juan, Francisco E-06071 Badajoz (ES)**

(74) Representative: **Carpintero Lopez, Francisco et al Herrero & Asociados, S.L. Alcalá 35 28014 Madrid (ES)**

(54) **UNIT, MODULAR SYSTEM AND METHOD FOR MEASURING, PROCESSING AND REMOTELY MONITORING ELECTRICAL BIOIMPEDANCE**

(57)     The measuring and processing unit (20) of the invention comprises an electrical bioimpedance measuring sub-unit (9) and a processing sub-unit (10) for the discrete processing of measurements including a memory element for storing the measurements.

The modular electrical bioimpedance measuring, processing and monitoring system comprises at least one of the aforementioned bioimpedance measuring and processing units (20). The modular system allows transmitting measurements to an external unit (30), such as a computer or PDA, for processing and/or monitoring.

The bioimpedance data can be transmitted over the Internet or a GSM network from the external unit (30) to a remote unit for remote monitoring.

FIG. 1

EP 2 752 158 A1

**Description**

Object and Technical Field of the Invention

The present invention relates to electrical impedance or bioimpedance measurement, particularly in a biological material; an animal or plant tissue; part of or the entire human or animal body, of an organism or of a manufactured product of animal or plant origin.

The bioimpedance measurement includes the bioimpedance spectrometry technique, whereby the alternating excitation current is scanned in a specific frequency range.

The object of the invention is to provide a bioimpedance measuring and measurement processing unit. Another object of the invention is to provide a bioimpedance measuring system, for processing and monitoring measurements, that allows integrating a plurality of said units in a modular manner, in cooperation with an external measurement monitoring unit, such as a computer or personal digital assistant (PDA) in particular. The final object of the invention is to provide a method which allows remotely monitoring the measurements by means of transmitting said measurements over the Internet, a GSM network or the like to a remote processing unit.

The invention essentially applies in the fields of medicine and food technology.

Background of the Invention

The continuously increasing life expectancy of people in developed countries is causing population aging. As a result, the ever-increasing segment of elderly population requires progressively more extensive health care, including preventive health care, and not only in hospitals, but rather in their own homes (home monitoring), which, however, reduces the number of health center visits.

On the other hand, the higher quality of life demanded by people in general requires conducting preventive examinations intended for the early detection of health problems, where the chances of success of different therapies increase significantly while at the same time costs are reduced.

The preceding considerations have an increasing repercussion on the resources required by public health systems in developed countries. However, the reality often faced by said public health systems is the exact opposite, such that they must face greater demands with fewer resources.

In the field of food technology, there are processes used for manufacturing products essentially of animal origin involving changes in the tissue or biological material cell structure, such as the meat product maturation or curing process, for example. In many cases, these processes still preserve a substantial artisanal component that has not favored progress in product quality standardization and, ultimately, the penetration of such products on demanding markets which, in turn, are those which have the greatest purchasing power.

On the other hand, most methods used in the industry for determining food properties (composition) are destructive, rendering the part under study useless. Designing non-destructive measuring systems is a challenge. However, in addition to preserving the integrity of the analyzed samples, new analytical techniques in demand must have a low cost and a simple operation that does not depend on skilled labor, or even more so, the possibility of the consumer him/herself carrying out certain controls is of great interest.

The significant progress experienced in the microelectronics and telecommunications industries over recent years, as well as the reduction of the corresponding associated costs, are opening up great prospects for providing satisfactory solutions to problems such as the foregoing.

Under the term electrical bioimpedance, the underlying concept is the opposition which organisms, tissues and biological materials have in general against the passage of an alternating electric current. The intensity of the current injected into tissues is so low that it does not stimulate excitable tissues, which allows using it in the health field in patients with different conditions, and even in newborns.

As is well known, electrical impedance is generally a complex quantity and in the particular case of bioimpedance, the real part represents the resistance of intra- and extracellular fluids, and the phase, by contrast, reflects temporary charge retention in cell membranes, in a manner similar to what occurs in capacitors. Accordingly and depending on their composition, biological tissues act to a greater or lesser extent like conductors or dielectrics, and said composition can be derived from the electrical (bioimpedance) characterization.

In relation to the frequency of the alternating excitation current, the bioimpedance technique can be applied using a single frequency, generally of 50 KHz, which is a frequency at which the current flows in a weighted manner through the intra- and extracellular fluids. In contrast with the foregoing, the bioimpedance spectrometry technique involves scanning the signal in a specific frequency range of the excitation current with a specific resolution.

Electrical bioimpedance is therefore not a physiological variable, but rather a technique which allows characterizing tissues and biological materials through their electrical behavior.

Today, the application of electrical bioimpedance technique to determining the global composition of the human body, to diagnosing certain diseases, and generally, to controlling food industry processes or for determining food compositions, is well known.

Conventionally, the electrical bioimpedance technique has pros and cons. The positive aspects that can be highlighted include the fact that it is a simple, non-invasive, non-destructive, non-ionizing and relatively cost-effective technique. With respect to the negative aspects, the technique is not very precise, fundamentally in applications intended for medical diagnosis, particularly compared with other existing alternatives (X-rays, computerized axial tomography, nuclear magnetic resonance, etc.), even though these alternatives are invasive and costly techniques that cannot be used repeatedly). Furthermore, the electrical bioimpedance technique lacks standardized and quality control methods.

The first applications of bioimpedance analysis were aimed at determining global body composition (fat-free mass, intra- and extracellular water content, etc.). A number of commercially available apparatus carrying out such general analysis of the body composition are known, even though all of them use indirect adjustment methods for determining said parameters based on bioimpedance measurements. These indirect adjustment methods are based on deriving the fat, muscle and water contents, for example, from the results of the bioimpedance measurements, depending on anthropometric parameters such as weight, height, gender, age, etc.

Other known applications of the bioimpedance technique aimed at medical diagnosis are, for example: determination of hidden abdominal fat, detection of various types of cancer, liver impairments, caries, nutritional status, osteoarthritis, tendon inflammation, etc. In these cases, the bioimpedance analysis is carried out in well-localized areas of the body.

Generally, apparatus for measuring electrical bioimpedance, essentially those which are applied to medical diagnosis, are based on relatively complex architectures. This complexity means that these apparatus are normally restricted for use only in hospitals and health care centers, being able to be handled only by skilled personnel.

The bioimpedance technique is also very useful in the field of food technology. Bioimpedance is particularly a technique of interest for controlling those food manufacturing processes having associated structural changes at the cellular level and for food composition quality control. Another objective to be achieved in this industrial sector due to commercial interest is the development of cost-effective, portable and easy-to-use devices with non-destructive associated methods that are capable of evaluating food composition.

In view of the foregoing in relation to the known state of the art, it would therefore be desirable to have a unit, system, apparatus or equipment for measuring, processing and monitoring bioimpedance, that brings together the advantages of simplicity, operation simplicity and portability with very small dimensions, such that it can be handled by the patient him/herself or by any layperson. Likewise, it would also be desirable for one and the same system to serve for various applications both in the medical field and food technology field.

Description of the Invention

In order to solve the described technical problem and to improve known bioimpedance measuring, processing and monitoring units and systems, the proposed invention provides the technical features and effects described below.

The electrical bioimpedance measuring and processing unit according to the invention is characterized in that it comprises a bioimpedance measuring sub-unit and a processing sub-unit including a memory element for storing measurements. The bioimpedance measurements are performed on a body, such as, particularly, a biological material, animal or plant tissue, part of or the entire human or animal body, of an organism or of a manufactured product of animal or plant origin, etc., being applicable in several fields.

The memory element is arranged in the processing sub-unit such that it enables the sub-unit to supply the measurements discretely. For the purposes of the present specification, "supply measurements discretely" is understood as the measurements being stored in the memory element of the unit until the supply thereof, for example, until the time in which they are transmitted to an external or remote processing or monitoring unit, as will be explained below. This feature and functionality of the invention for supplying measurements discretely allows providing an impedance measuring system which is considerably simpler and easier to use than known systems, and it is also highly portable since the components and devices conventionally necessary for assuring a supply of measurements in a continuous manner can be dispensed with.

In an embodiment of the electrical bioimpedance measuring sub-unit according to the invention, said sub-unit comprises a bioimpedance measuring device including the following elements: an alternating current generator, a bioimpedance voltage measuring circuit, a reference voltage measuring circuit and a magnitude and phase detection circuit. The alternating current generator is electrically coupled to a current electrode ($I^+$) through which the electric current is injected and to a current electrode ($I^-$) through which the current is collected, serving to provide an excitation current between the current electrodes in contact with the body. This current is likewise forced to flow through a reference impedance. The bioimpedance voltage measuring circuit is electrically coupled to a positive bioimpedance electrode ($V^+$) and to a negative bioimpedance electrode ($V^-$), serving to obtain the voltage drop of the excitation current between the bioimpedance electrodes in contact with the body. The reference voltage measuring circuit serves to obtain the voltage drop of the excitation current in the reference impedance. Finally, the magnitude and phase detection circuit serves to obtain the modulus ratio and phase difference between the voltage obtained by the bioimpedance voltage measuring circuit and the voltage obtained by the reference voltage measuring circuit, which allows obtaining the measurements to be processed.

Bioimpedance is measured by arranging the four electrodes such that one of the current electrodes (I⁺) injects the alternating excitation current into the body and the other (I⁻) collects it, respectively, and the two remaining bioimpedance electrodes (V⁺ and V⁻) are also respectively fixed to the points between which the voltage difference is measured.

The alternating current generator is preferably configured for generating an alternating current having amplitude of about 10 $\mu$A and in the frequency range comprised between 1 KHz and 1 MHz, with an adjustable resolution. The intensity of the current injected into the tissues is low enough so as to not stimulate the excitable tissues, which allows using it in the health field in patients with different conditions, and even in newborns. On the other hand, relatively high frequencies allow greater signal propagation inside the body, which makes obtaining information about the composition of the deepest areas inside the body possible.

In a variant of an electrical bioimpedance measuring sub-unit, the bioimpedance voltage measuring circuit and the reference voltage measuring circuit of the bioimpedance measuring device can comprise, respectively, a bioimpedance voltage instrumentation amplifier and a reference voltage instrumentation amplifier that are nominally identical.

The detection circuit, responsible for obtaining two DC voltage levels from which it determines the modulus and phase of the complex electrical bioimpedance, performs the calculation according to the phase difference and the ratio between the magnitudes of two electrical signals.

Each instrumentation amplifier can be formed as an amplifier circuit comprising a cascade of voltage-to-current and current-to-voltage conversion stages. Therefore, the common-mode voltage dependence between the input and output terminals of the amplifier can be eliminated, reducing power consumption and additionally improving the signal-to-noise ratio. Particularly, each amplifier can be a two-stage amplifier with a first voltage-to-current conversion stage and a second current-to-voltage conversion stage.

Complementarily, the bioimpedance measuring device can comprise a bioimpedance voltage rectifier circuit and a reference voltage rectifier circuit. A bioimpedance voltage rectifier circuit can be coupled between the bioimpedance voltage measuring circuit and the detection circuit, such that it allows transforming the voltage obtained by said measuring circuit into electrical signals from which the value of the modulus and of the phase of said voltage obtained by said measuring circuit can be directly extracted. A reference voltage rectifier circuit coupled between the reference voltage measuring circuit and the detection circuit can similarly be used. The bioimpedance measuring device preferably comprises a bioimpedance voltage rectifier circuit coupled between the bioimpedance voltage measuring circuit and the detection circuit, and a reference voltage rectifier circuit coupled between the reference voltage measuring circuit and the detection circuit. The bioimpedance voltage rectifier circuit allows transforming the voltage obtained by the bioimpedance voltage measuring circuit into a first electrical square signal, having a phase equivalent to the phase of said voltage obtained by said measuring circuit, and into a second electrical signal having a DC voltage level equivalent to the modulus of said voltage obtained by said measuring circuit. On the other hand, the reference voltage rectifier circuit allows transforming the voltage obtained by the reference voltage measuring circuit into a third electrical square signal, having a phase equivalent to the phase of said voltage obtained by said measuring circuit, and into a fourth electrical signal having a DC voltage level equivalent to the modulus of said voltage obtained by said measuring circuit. For these purposes, the term "equivalent" means that the amplitude and phase of the electrical signals are defined and determined according to the magnitude and phase of the corresponding voltage signals, said function being nominally identical for the case of the bioimpedance voltage rectifier circuit and for the case of the reference voltage rectifier circuit. For example, an equivalent electrical signal can be formed by a DC voltage signal that is proportional to the modulus or phase of the voltage obtained by the measuring circuit.

Said bioimpedance voltage rectifier circuit and reference voltage rectifier circuit can be formed respectively by means of a bioimpedance voltage logarithmic amplifier and a reference voltage logarithmic amplifier that are nominally identical.

It is also contemplated that the processing sub-unit additionally includes a control device coupled to the memory element, the control device comprising a circuit for selecting the type of bioimpedance measurement. "Type of bioimpedance measurement" is understood as the selection of the amplitude and frequency of the excitation current, or the amplitude and frequency range in the event of a bioimpedance spectrometry analysis.

In a preferred embodiment of the invention, the processing sub-unit comprises a transmission/reception device coupled to the control device for transmitting the measurements stored in the memory element to an external unit, such as a computer or PDA in particular. Likewise, the processing sub-unit can be configured for receiving and processing data and/or orders about the type of measurement to be taken from the external unit. For such purpose, the measuring unit transmission/reception device can be based on a wireless communication protocol, incorporating to that end a radio frequency communication circuit and an antenna. Complementarily, an external unit transmission/reception device can be arranged associated with the external unit, for example said device can be removable from and connected to the external device through USB, acting as an interface for transmitting the orders about the type of measurement and receiving measurements between the bioimpedance measuring and processing unit and the external unit.

Alternatively or complementarily, the measurement processing sub-unit can include an LCD screen coupled to the control device and configured for displaying measurements and/or displaying information about the type of measurement, etc.

The bioimpedance measuring device can be manufactured with an application-specific integrated circuit customized for complementary metal-oxide-semiconductor (CMOS) technology. Current manufacturing techniques offer lengths of 0.35 $\mu$m and less. Improvement in manufacturing technologies has in turn increased production output, which allows manufacturing integrated circuits in which several functional blocks are included at an increasingly more cost-effective price. Therefore, the physical dimensions of the bioimpedance measuring and processing unit can be very small, having an also very low power consumption that assures long-lasting operation using a microbattery. At the same time, the integration of all the functional blocks forming the measuring and processing unit into a single integrated circuit favors the manufacture of nominally identical blocks. This is achieved by using microelectronics design techniques that reduce the impact of variations on CMOS device manufacturing parameters.

On the other hand, the present invention relates to a modular bioimpedance measuring, processing and monitoring system, characterized in that it comprises at least one bioimpedance measuring and processing unit of the type described above according to the invention.

When the specific application comprises several bioimpedance units, one of them can act as a coordinator (coordination unit) of a wireless network for transmitting measurements to an external unit, for processing and/or monitoring in said external unit. The functions of the coordination unit include the following: receiving measurements from the measuring and processing units, transmitting measurements to the external unit, and/or receiving the selection of the type of measurement from the external unit and transmitting same to the measuring and processing units. Both the case where the coordination unit integrates a measuring and processing unit and the case where the coordination unit is only configured to perform said coordination functions are contemplated. The results of the measurements are stored in the memory element of the measuring or processing units, and/or of the corresponding coordination unit of the system if the application includes more than one. The measurements are preferably transmitted to the external unit when the external unit and the coordination unit detect one another. The bioimpedance data can then be transmitted over the Internet or a GSM network from the external unit to a remote unit which processes and stores the data remotely, fundamentally in the case of health-related applications, or be processed and/or monitored directly in the external unit, which may be sufficient for other applications of the proposed system. The remote processing unit can also be a computer or PDA in particular.

Finally, a possible embodiment of the modular system for applications in medical diagnosis according to the invention comprises a bioimpedance measuring strap which is arranged in contact with the human body for taking the bioimpedance measurement. Said strap can be a belt (around the waist) or an armband (around the arm or leg), for example, the current electrodes and the impedance electrodes being arranged on the face thereof in contact with the body. This modular system embodiment is characterized in that the current electrodes (I$^+$ and I$^-$) of each bioimpedance measuring and processing unit, as well as the respective bioimpedance electrodes (V$^+$ and V$^-$), are arranged sufficiently spaced from one another so that the bioimpedance measurement suitably reflects the contribution of an inner part of the body with respect to the contribution of a surface part thereof.

Brief Description of the Drawings

To complement the explanation of the invention and for the purpose of aiding to better understand its technical features, the rest of this specification refers to the attached drawings in which an embodiment of the device of the invention has been depicted by way of non-limiting, practical example.

In said drawings:

Figure 1 schematically shows a block diagram of a bioimpedance measuring and processing unit according to the invention.

Figure 2 depicts a modular bioimpedance measuring and processing system according to the invention, having three bioimpedance measuring and processing units. Figure 2 also depicts an external unit (computer) associated with the modular bioimpedance measuring and processing system, for monitoring the measurements taken by same.

Figure 3 shows an embodiment for a specific application within the medical field such as the measurement of visceral fat, a bioimpedance measuring belt that is place around the waist of a human body. The bioimpedance measuring belt shown has two bioimpedance measuring and processing units.

The references used in the drawings are as follows:

1: body
2: alternating current generator
3: reference impedance
4: bioimpedance voltage instrumentation amplifier
5: reference voltage instrumentation amplifier
6: bioimpedance voltage logarithmic amplifier
7: reference voltage logarithmic amplifier
8: magnitude and phase detection circuit
9: bioimpedance measuring sub-unit

| | |
|---|---|
| 10: | measurement processing sub-unit |
| 11: | control device |
| 12: | transmission/reception device |
| 13: | antenna |
| 20: | bioimpedance measuring and processing unit |
| 21: | coordination unit |
| 30: | external unit |
| 31: | external unit transmission/reception device |
| 40: | bioimpedance measuring belt for measuring visceral fat The symbols used in the drawings are as follows: |
| $I^+$: | current injection electrode |
| $I^-$: | current collection electrode |
| $V^+$: | positive bioimpedance electrode |
| $V^-$: | negative bioimpedance electrode |
| R: | reference resistance |
| Z: | electrical bioimpedance |
| $V_m$: | voltage proportional to modulus ratio |
| $V_p$: | voltage proportional to phase difference |

Detailed Description of an Embodiment

An embodiment of the invention is described in detail below in reference to the drawings.

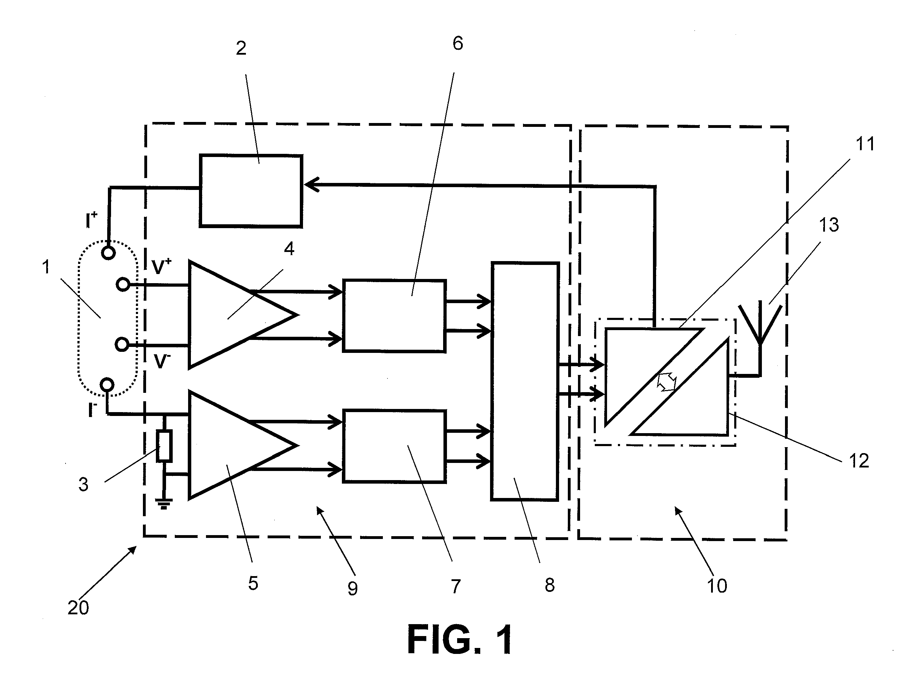

Figure 1 corresponds to a simplified block diagram of the measuring and processing unit (20) carrying out the bioimpedance measurement, particularly the bioimpedance spectrometry, powered by a microbattery (not depicted) encapsulated in the unit. It has two well differentiated parts (9, 10). The first bioimpedance measuring sub-unit (9) is responsible for taking the measurements of the modulus and phase of the bioimpedance of a body (1) in contact with the four electrodes ($I^+$, $V^+$, $I^-$, $V^-$) of the unit (20), as can be seen. Said measuring sub-unit (9) was specifically designed for the application and manufactured in CMOS technology. The second measurement processing sub-unit (10) is responsible for communicating with an external unit or another measuring or processing unit. One variant of the embodiment of the measuring and processing unit (20) can consist of replacing the transmission/reception device (12) with an LCD screen useful for those applications in which the transmission of the results of the measurements is not required and only viewing said results is required.

In the illustrated embodiment, the measuring sub-unit (9) comprises the contacts of four electrodes ($I^+$, $V^+$, $I^-$, $V^-$). The alternating current responsible for stimulating the body (1) the complex impedance Z of which is to be known, existing between the contact points of the bioimpedance electrodes ($V^+$, $V^-$), is injected between the electrodes ($I^+$, $I^-$). This current is likewise forced to flow through a resistive reference impedance or reference resistance (3) with a known value R. The voltage drop between the two points of the body (1) where the electrodes ($V^+$, $V^-$) are in contact is simultaneously collected.

An alternating current generator (2) is responsible for injecting the small excitation current, circulating it between the current electrodes ($I^+$, $I^-$) with an amplitude of 10 μA. To perform spectrometry analysis, the current generator (2) is configured so that it is capable of scanning in a frequency range comprised between 1 kHz and 1 MHz. Nevertheless, the variation of the frequency of the excitation current can be programmed within another different range.

The electrical signals collected by the electrodes ($V^+$, $V^-$) are in turn input signals for a first bioimpedance voltage instrumentation amplifier (4) extracting and amplifying the voltage in the body. A second reference voltage instrumentation amplifier (5) simultaneously collects the electrical voltage between the ends of the reference resistance (3) and amplifies this signal by the same factor as the instrumentation amplifier.

The instrumentation amplifiers (4, 5) are nominally identical and can be designed to operate in a completely differential mode, such that they deliver the coded signal as the difference of two counter-phase signals existing in each of the output terminals, respectively, thus improving the signal-to-noise ratio. They have a two-stage structure. The first stage amplifies and converts the electrical alternating voltage signals at the input into electrical alternating current signals. The second stage converts these electrical current signals back into electrical alternating voltage signals. The common-mode voltage dependence in the input and output terminals of the instrumentation amplifier can thus be eliminated, which favors the reduction of minimal supply voltage of this cell and, therefore, a considerable reduction in power consumption. Furthermore, by using both stages a high rejection of the common-mode component of the input signals is achieved in order to limit the influence of parasitic impedances present on the contact surface between the electrodes and the body.

The differential output signals of the instrumentation amplifiers (4, 5) in turn form the input signals of two bioimpedance voltage and reference voltage logarithmic amplifiers (8, 9), respectively, which are also nominally identical to one another. Both logarithmic amplifiers operate on the input signal in differential configuration and each of them provides a simple direct voltage output which is a logarithmic function of the input signal. Likewise, they provide an alternating voltage signal with a square wave and having the same frequency as the voltage signal present in the input terminals thereof.

In order to obtain two direct voltage signals representing the magnitude and phase of the bioimpedance associated with the body (1) between the electrodes ($V^+$ and $V^-$), and the frequency determined by the frequency of the excitation current generated in the generator (2), the output signals of the logarithmic amplifiers (6, 7) are in turn the inputs of a magnitude and phase detection circuit (8). The detection circuit (8) in turn has two circuits. One of them is dedicated to generating a signal proportional to the phase difference, Vp, between the alternating voltage signals provided by the logarithmic amplifiers (6, 7). In contrast, the second circuit does likewise with the logarithm of the modulus ratio, $V_m$, of the voltage signals provided by the instrumentation amplifiers (4, 5). Therefore and by using phasor notation, the value of bioimpedance Z coincides with the following expression:

$$Z = |Z| \cdot e^{j\phi} = R \cdot 10^{Vm} \cdot e^{jVp}$$

where R is the value of the reference resistance (3) and $\phi$ is the phase of the electrical bioimpedance.

As explained above, the purpose of the processing sub-unit (10) is to control the measuring sub-unit, process the measurements and transmit/receive the data associated with the modulus and phase of the bioimpedance for each of the frequencies of the alternating excitation current generated in (2), using a wireless communication protocol. Said sub-unit (10) in turn has a control device (11) and a measuring unit transmission/reception device (12), including an antenna (13).

The control device (11) performs the following functions:

- Selecting the frequency of the alternating current signal within the range of 1 kHz - 1 MHz provided by the generator (2).
- Controlling the analog-to-digital conversion mode according to the chosen communication protocol.
- Storing data associated with the modulus and phase of the bioimpedance in a memory block. These values can therefore be stored until an external unit requires the measurements, for example.

For a practical embodiment of the processing sub-unit (10), an ATmega128 micro-controller incorporating an analog-to-digital conversion stage and a memory element can be selected as a control device (11); a commercial CC2420 module which operates using the ZigBee protocol in the 2.4 GHz band can be used as a measuring unit transmission/reception device (12).

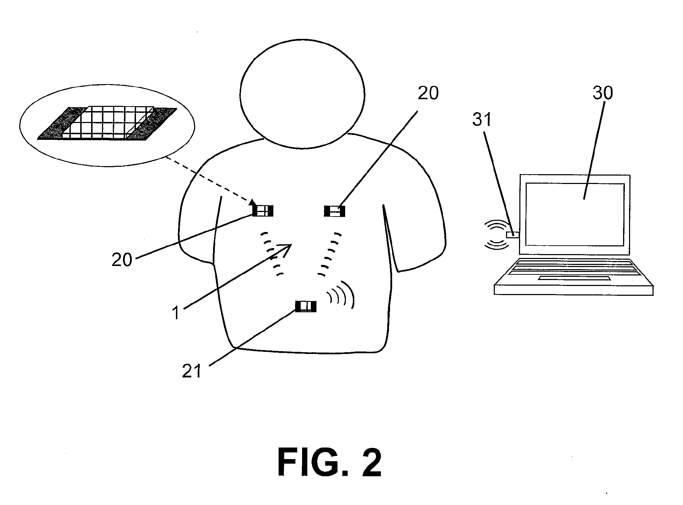

In reference to Figure 2, this figure shows a conceptual diagram of the modular bioimpedance measuring, processing and monitoring system according to the invention. A network formed by three bioimpedance measuring and processing units (20), one of which is a coordination unit (21), has been arranged therein, on the surface of a body, in this case a human body, all with the same circuit structure shown in Figure 1. Each unit (20) is provided with four electrodes which, for the sake of simplicity of the drawing, are presumably arranged on the face contacting the skin of the body, and takes the bioimpedance measurement in the areas where they contact the skin, respectively. The bioimpedance measurements obtained by the measuring and processing units (20) are transmitted to the coordination unit (21) through a wireless communication protocol. Said measurements, together with measurements taken by the coordination unit (21) itself, are stored in the memory element of said unit until the external unit transmission/reception device (31) connected through a Universal Serial Port (USB) to the external unit (30) is detected. At this moment the results of the bioimpedance measurements recorded by all the units (20, 21) forming the network are transmitted together with the identification of each of them with the corresponding data. Said data is stored and/or processed in the external unit (30) or simply transmitted to another remote server over the Internet or a GSM network. The coordination unit (21) can also be communicated with a PDA and the data is transmitted from the PDA to the remote processing unit over the Internet or a GSM network.

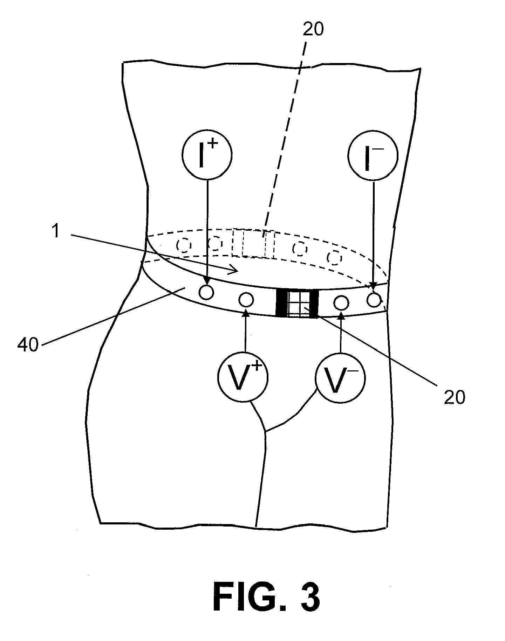

Finally, as an example of an application of the proposed modular system, Figure 3 illustrates the possible arrangement of a network of bioimpedance measuring and processing units in the context of its application for evaluating hidden fat or visceral fat in an individual. The objective of the application of the modular system according to the embodiment in the field of health is to allow a precise evaluation of visceral fat surrounding the internal organs by means of using more than one electrical pathway. In this case, a bioimpedance measuring belt (40) which is placed around the waist of an individual at the height of his/her navel is included. In this particular case, said belt (40) has two bioimpedance units (20). Each of the units (20) comprises the contacts of four electrodes ($I^+$, $V^+$, $I^-$, $V^-$). The electrodes responsible for injecting the excitation current ($I^+$, $I^-$) are arranged at a sufficient distance so that the bioimpedance measurement reflects a greater contribution of hidden fat, not subcutaneous fat.

It is not considered necessary to further elaborate on this description so that any person skilled in the art can comprehend the scope of the invention and advantages derived from it. Particularly, for the manufacture of different components, the manufacturing processes and the necessary materials are known in the technical field and fully accessible. Generally, the shape, size and arrangement of the elements may vary provided that it does not alter the essential features of the invention.

**Claims**

1. Electrical bioimpedance measuring and processing unit for measuring and processing the electrical bioimpedance of a body such as, particularly, a biological material, an animal or plant tissue, part of or the entire human or animal body, of an organism or of a manufactured product of animal or plant origin; **characterized in that** it comprises:

   a bioimpedance measuring sub-unit for measuring the bioimpedance of the body; and
   a processing sub-unit for the discrete processing of measurements comprising a memory element for storing the measurements.

**2.** Electrical bioimpedance measuring and processing unit according to claim 1, **characterized in that**:

> the bioimpedance measuring sub-unit comprises a bioimpedance measuring device including:
>
>> - an alternating current generator electrically coupled to a current injection electrode ($I^+$), to a current collection electrode ($I^-$) and to a reference impedance, for generating an excitation current circulating between the current electrodes in contact with the body and through the reference impedance;
>> - a bioimpedance voltage measuring circuit electrically coupled to a positive bioimpedance electrode ($V^+$) and to a negative bioimpedance electrode ($V^-$), for obtaining the voltage drop of the excitation current between the bioimpedance electrodes in contact with the body;
>> - a reference voltage measuring circuit for obtaining the voltage drop of the excitation current in the reference impedance; and
>> - a magnitude and phase detection circuit for obtaining the modulus ratio ($V_m$) and phase difference (Vp) between the voltage obtained by the bioimpedance voltage measuring circuit and the voltage obtained by the reference voltage measuring circuit.

**3.** Electrical bioimpedance measuring and processing unit according to claim 2, **characterized in that** the bioimpedance voltage measuring circuit and the reference voltage measuring circuit of the bioimpedance measuring device respectively comprise a bioimpedance voltage instrumentation amplifier and a reference voltage instrumentation amplifier that are nominally identical.

**4.** Electrical bioimpedance measuring and processing unit according to claim 3, **characterized in that** each instrumentation amplifier is formed by a cascade of voltage-to-current and current-to-voltage conversion stages; such that the common-mode voltage dependence between the input and output terminals of the amplifier is eliminated, reducing power consumption and improving the signal-to-noise ratio.

**5.** Electrical bioimpedance measuring and processing unit according to one of claims 2-4, **characterized in that** the bioimpedance measuring device additionally comprises:

> - a bioimpedance voltage rectifier circuit coupled between the bioimpedance voltage measuring circuit and the detection circuit, for transforming the voltage obtained by said measuring circuit into a first electrical square signal having a phase equivalent to the phase of said voltage obtained by said measuring circuit, and into a second electrical signal having a DC voltage level equivalent to the modulus of said voltage obtained by said measuring circuit;
- a reference voltage rectifier circuit coupled between the reference voltage measuring circuit and the detection circuit, for transforming the voltage obtained by said measuring circuit into a third electrical square signal having a phase equivalent to the phase of said voltage obtained by said measuring circuit, and into a fourth electrical signal having a DC voltage level equivalent to the modulus of said voltage obtained by said measuring circuit.

**6.** Electrical bioimpedance measuring and processing unit according to any one of claims 1-5, **characterized in that** the processing sub-unit additionally includes a control device coupled to the memory element, the control device comprising a circuit for selecting the type of bioimpedance measurement.

**7.** Electrical bioimpedance measuring and processing unit according to claim 6, **characterized in that** the processing sub-unit includes an LCD screen coupled to the control device, the LCD screen configured to perform a function selected from the group consisting of: displaying measurements, displaying information about the type of measurement and a combination of the preceding functions.

**8.** Electrical bioimpedance measuring and processing unit according to any one of claims 6-7, **characterized in that** the processing sub-unit comprises a transmission/reception device coupled to the control device and configured to perform a function selected from the group consisting of: transmitting measurements to an external unit, such as a computer or PDA in particular; receiving and processing orders about the type of measurement from the external unit; and a combination of the preceding functions.

**9.** Electrical bioimpedance measuring and processing unit according to claim 8, **characterized in that** the transmission/reception device comprises a radio frequency communication circuit and an antenna.

**10.** Electrical bioimpedance measuring and processing unit according to any one of claims 1-9, **characterized in that** the bioimpedance measuring device complies with CMOS technology.

**11.** Modular electrical bioimpedance measuring, processing and monitoring system, **characterized in that** it comprises at least one bioimpedance measuring and processing unit defined in any one of claims 1-10.

**12.** Modular electrical bioimpedance measuring, processing and monitoring system comprising at least one bioimpedance measuring and processing unit according to any one of claims 10-11, **characterized in that** it comprises a coordination unit configured to perform a function selected from the group consisting of: receiving measurements from the measuring and processing units, transmitting measurements to the external unit, receiving the selection of the type of measurement from the external unit and transmitting same to the measuring and processing units, and a combination of the preceding functions.

**13.** Modular electrical bioimpedance measuring, processing and monitoring system according to claim 12, **characterized in that** the coordination unit is integrated in one of the bioimpedance measuring and processing units.

**14.** Modular electrical bioimpedance measuring, processing and monitoring system according to one of claims 11-13, comprising a bioimpedance measuring strap which is arranged in contact with the body for taking bioimpedance measurement, the strap being particularly formed with a belt or an armband, the current electrodes and the impedance electrodes being arranged on the face thereof in contact with the body; **characterized in that** the positive and negative current electrodes of each bioimpedance measuring and processing unit, as well as the respective positive and negative bioimpedance electrodes are arranged sufficiently spaced from one another so that the bioimpedance measurement suitably reflects the contribution of an inner part of the body with respect to the contribution of a surface part thereof.

**15.** Method for measuring, processing and remotely monitoring electrical bioimpedance, **characterized in that** it comprises the following steps:

  - obtaining bioimpedance measurements with a modular measuring, processing and monitoring system defined in any one of claims 11-14; and
  - transmitting the measurements over the Internet, a GSM network or the like to a remote processing unit, such as a computer or PDA in particular.

**FIG. 1**

# FIG. 2

# FIG. 3

# INTERNATIONAL SEARCH REPORT

| International application No. |
| --- |
| PCT/ES2012/070635 |

**A. CLASSIFICATION OF SUBJECT MATTER**

*A61B5/053* (2006.01)

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

EPODOC, INVENES

**C. DOCUMENTS CONSIDERED TO BE RELEVANT**

| Category* | Citation of document, with indication, where appropriate, of the relevant passages | Relevant to claim No. |
| --- | --- | --- |
| X | WO 2010104952 A2 (CORVENTIS INC ET AL.) 16/09/2010, paragraphs[0013 - 0059]; paragraphs[0104 - 243]; figure 1, | 1-15 |
| X | US 2006184060 A1 ( BELALCAZAR ANDRES ET AL.) 17/08/2006, paragraphs[0002 - 0026], paragraphs[0060 - 0069]; figure 11 | 1-15 |
| A | WO 2008029316 A2 (PHILIPS INTELLECTUAL PROPERTY ET AL.) 13/03/2008, pages 1 - 7; figure 4, | 1-15 |
| A | US 2004152996 A1 (GERSING EBERHARD ) 05/08/2004, paragraphs[0005 - 0059]; | 1-15 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 12/12/2012 | **(18/12/2012)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> A. Casado Fernández <br><br><br> Telephone No. 91 3496871 |

Form PCT/ISA/210 (second sheet) (July 2009)

## INTERNATIONAL SEARCH REPORT

Information on patent family members

| International application No. |
|---|
| PCT/ES2012/070635 |

| Patent document cited in the search report | Publication date | Patent family member(s) | Publication date |
|---|---|---|---|
| WO2010104952 A | 16.09.2010 | NONE | |
| US2006184060 A | 17.08.2006 | US7447543 B | 04.11.2008 |
| | | WO2006088805 A | 24.08.2006 |
| | | EP1848334 A | 31.10.2007 |
| | | EP20060734952 | 14.02.2006 |
| | | CN101163443 A | 16.04.2008 |
| | | CN100584270 C | 27.01.2010 |
| | | JP2008529678 A | 07.08.2008 |
| | | HK1119550 A | 15.10.2010 |
| WO2008029316 A | 13.03.2008 | NONE | |
| US2004152996 A | 05.08.2004 | WO02094090 A | 28.11.2002 |
| | | DE10125359 AB | 12.12.2002 |
| | | EP1389946 AB | 25.02.2004 |
| | | EP20020738099 | 23.05.2002 |
| | | AT522172 T | 15.09.2011 |

Form PCT/ISA/210 (patent family annex) (July 2009)